# EUROPEAN PATENT APPLICATION

(11) **EP 4 039 788 A1**
(43) Date of publication of application: **10.08.2022**
(21) Application number: 21155752.5
(22) Date of filing: 08.02.2021
(51) Int. Cl.: C12M 1/32, C12M 3/06, C12M 1/00, C12M 1/12

(54) **CELL CULTURE MEMBRANE STRUCTURE, METHODS FOR PRODUCING THE SAME, CELL CULTURE PLATE AND MICROFLUIDIC DEVICE USING THE SAME**

(71) Applicant: Finnadvance Oy, 90220 Oulu (FI)
(72) Inventor: Singh, Prateek, 90130 Oulu (FI); Nguyen, Tuan, 90130 Oulu (FI); Nurmi, Tuomas, 90240 Oulu (FI)
(74) Representative: Papula Oy

(57) **Abstract**

The present disclosure refers to a porous membrane structure that can be used as a cell culture support adapted for specific cells to be grown thereon. The porous membrane structure comprises a membrane having through pores formed therein and at least one coating layer provided on the membrane such that the through pores remain open. Said adaptation is provided by selecting membrane and coating materials based on at least one type of cells to be grown on the at least one coating layer. By so doing, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells.

## Description

### TECHNICAL FIELD

The present disclosure relates generally to the field of biotechnology. In particular, the present disclosure relates to a cell culture membrane structure, methods for producing this structure, as well as a cell culture plate and a microfluidic device using this structure.

### BACKGROUND

Cell culture or cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells *in vivo.* This may be done by performing the cell culture in large vessels, such as dishes, spinner and shaker flasks, etc. However, the cell culture conditions provided by these vessels do not truly represent the *in vivo* environment of the cells being cultured. Moreover, since these vessels have a large volume, they consume significant amounts of reagents, culture media, chemicals, etc., thereby making it difficult to control and/or alter the cell culture conditions. On top of that, these vessels are generally unable to provide uniform cell culture conditions to all the cells throughout the volume of the vessel.

At least some of the above-listed drawbacks may be eliminated by using thin, porous membranes as cell culture supports. In particular, the porous membrane provides improved control over the cell culture conditions. The porous membranes also enable parallel culturing of a large number of cells under the same or similar cell culture conditions. In general, the porous membranes enable the partitioning of cellular microenvironments and cell types in *vitro,* while still allowing physical and biochemical crosstalk between cells.

However, the limiting factor of using the thin porous membranes is that they need to be strong enough to withstand forces applied thereto during their production and subsequent manipulations. At the same time, the room for adjusting membrane properties for the cell culture is very limited. In many cases, the porous membranes cannot be adapted for a specific cell culture.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a cell culture membrane structure that is adapted for specific cells to be grown thereon.

The objective above is achieved by the features of the independent claims in the appended claims. Further embodiments and examples are apparent from the dependent claims, the detailed description and the accompanying drawings.

According to a first aspect, a cell culture membrane structure is provided. The cell culture membrane structure comprises a membrane made of a membrane material. The membrane comprises through pores. The cell culture membrane structure further comprises at least one coating layer provided on the membrane such that the through pores of the membrane remain open. The at least one coating layer is made of a coating material. The membrane material and the coating material are selected based on at least one type of cells to be grown on the at least one coating layer. By altering the membrane and coating materials depending on cell types, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be grown thereon.

In one embodiment of the first aspect, the cell culture membrane structure further comprises chemical agents (e.g., antifouling agents, etc.) and/or biomolecules (e.g., antigens, proteins, carbohydrate, antibody molecules, etc.) bonded onto the at least one coating layer. By using the chemical agents and/or biomolecules, it is possible to provide an even larger spectrum of functionality for the cell culture membrane structure.

In one embodiment of the first aspect, the at least one coating layer comprises a first coating layer provided on a first side of the membrane and a second coating layer provided on a second opposite side of the membrane. By coating the membrane on either side, it is possible to additionally alter different physical and chemical properties of the cell culture membrane structure. For example, it is possible to alter elastic properties of the cell culture membrane structure mainly in a vertical direction (i.e. a direction perpendicular to the membrane) by using the coatings on either side.

In one embodiment of the first aspect, the coating material is the same or different for each of the first coating layer and the second coating layer. This may make it possible to study cell behavior on the cell culture membrane structure under different *in vitro* conditions.

In one embodiment of the first aspect, each of the first coating layer and the second coating layer has a different thickness and/or stiffness. This may also make it possible to study the cell behavior on the cell culture membrane structure under different *in vitro* conditions.

In one embodiment of the first aspect, the at least one type of cells comprises a first type of cells to be grown on the first coating layer and a second type of cells to be grown on the second coating layer. This may allow one to study the behavior of different cells on the cell culture membrane structure.

In one embodiment of the first aspect, each of the through pores has a pore size varying within a predefined range of values. In this embodiment, the through pores are spaced from each other by a distance varying within the same predefined range of values. By varying the pore size and the distance between the pores within the same range of values, one may alter different physical and chemical properties of the membrane, thereby influencing the cell behavior on the cell culture membrane structure.

In one embodiment of the first aspect, the predefined range of values is selected based on the at least one type of cells to be grown on the at least one coating layer. This may also provide different physical and chemical properties of the membrane, thereby influencing the cell behavior on the cell culture membrane structure.

In one embodiment of the first aspect, the pore sizes of the through pores are selected to be less than cell sizes of the at least one type of cells to be grown on the at least one coating layer. This embodiment is useful when it is required to prevent cells from migrating between opposite sides of the membrane through the pores.

In one embodiment of the first aspect, the at least one type of cells comprises a first type of cells and a second type of cells which are both to be grown on one of the first coating layer and the second coating layer. This may allow one to study the behavior of different cells on the same side of the cell culture membrane structure.

In one embodiment of the first aspect, each of the through pores has a pore size varying within a predefined range of values. The predefined range of values is selected (e.g., based on sizes and/or elastic properties of cells of the both types) such that the through pores are configured to pass one of the first type of cells and the second type of cells therethrough and retain another of the first type of cells and the second type of cells. At the same time, such through pores may be spaced from each other by a distance varying within the same predefined range of values. By having such through pores in the membrane, it is possible to squeeze or filter cells of a certain type therethrough in situations when cells of two different types are initially grown on the first or second coating layers (i.e. on the same side of the membrane). After said filtering or squeezing, the membrane will have cells of one type on one side and cells of another type on another (opposite) side.

In one embodiment of the first aspect, the membrane material is selected from one of polypropylene, polycarbonate, polyethersulfone, polyethylene terephthalate, polyimide, polytetrafluoroethylene, silicone, silicon nitride. By selecting from these membrane materials, one may also alter different physical and chemical properties of the cell culture membrane structure, thereby extending the range of its application.

In one embodiment of the first aspect, the coating material is selected from one or more of silicone, polyacrylamide, PEG polymer, acrylamide, gelatin derivative, nanocellulose. By selecting from these coating materials, one may also alter different physical and chemical properties of the cell culture membrane structure, thereby extending the range of its application.

According to a second aspect, a cell culture plate is provided. The cell culture plate comprises a housing encompassing a plurality of wells. Each well is provided with a cell culture media and a replaceable insert configured to be immersed into the cell culture media. The insert comprises the cell culture membrane structure according to the first aspect. With such a cell culture plate, it is possible to grow cells of desired type(s) on a large amount of cell culture membrane structures simultaneously and under the same growing conditions.

According to a third aspect, a microfluidic device is provided. The device comprises a housing having at least one microfluidic flow channel formed therein. Each of the at least one microfluidic flow channel is configured to pass a fluid or cell culture media therethrough. The device further comprises at least one cell culture membrane structure according to the first aspect. Each of the at least one cell culture membrane structure is in contact with one of the at least one microfluidic flow channel. With such configuration, it is possible to use the microfluidic device for different biomimetic purposes, depending on the cells to be grown of the cell culture structure(s).

In one embodiment of the third aspect, the housing further comprises at least one top loaded chamber. In this embodiment, each of the at least one top loaded chamber is connected to corresponding one of the at least one microfluidic flow channel via corresponding one of the at least one cell culture membrane structure. Each top loaded chamber allows a user to add different particles under study (e.g., an organoid or spheroid) to the corresponding cell culture membrane structure, thereby allowing the user to mimic conditions at different anatomical points more accurately, in more detail and with greater precision.

In one embodiment of the third aspect, each of the at least one microfluidic flow channel is divided by one of the at least one cell culture membrane structure into two microfluidic flow subchannels. This embodiment is useful when it is required to study cell behavior on either side of the cell culture membrane structure.

According to a fourth aspect, a method for producing a cell culture membrane structure is provided. The method starts with the step of providing a membrane made of a membrane material. Then, the method proceeds to the step of forming through pores in the membrane. After that, the next step is initiated, in which at least one coating layer is applied on the membrane such that the through pores of the membrane remain open. The at least one coating layer is made of a coating material. The membrane material and the coating material are selected based on at least one type of cells to be grown on the at least one coating layer. By altering the membrane and coating materials depending on cell types, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be grown thereon.

In one embodiment of the fourth aspect, the step of forming is performed by using at least one of particle/fluid bombardment, etching and laser burning. These techniques may allow one to form the through pores in the membrane more efficiently.

In one embodiment of the fourth aspect, the step of applying comprises: applying the at least one coating layer on the membrane in a continuous manner; and clearing the through pores of the membrane by using etching and/or air or liquid pressurizing techniques. By so doing, it is possible to remove the coating material from the pores more efficiently.

In one embodiment of the fourth aspect, the step of applying is performed by using one of spin coating, blade coating, spray coating, immersion coating, brush coating, stamping, and electrospraying. These techniques may allow one to apply the coating layer(s) on the membrane more efficiently.

According to a fifth aspect, a method for producing a cell culture membrane structure is provided. The method starts with the step of providing a membrane made of a membrane material. Then, the method proceeds to the step of applying at least one coating layer on the membrane in a continuous manner. The at least one coating layer is made of a coating material. After that, the next step is initiated, in which through pores coming through the at least one coating layer and the membrane are formed. The membrane material and the coating material are selected based on at least one type of cells to be grown on the at least one coating layer. By altering the membrane and coating materials depending on cell types, it is possible to alter different properties of the cell culture structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be grown thereon.

In one embodiment of the fifth aspect, the step of applying is performed by using one of spin coating, blade coating, spray coating, immersion coating, brush coating, stamping, and electrospraying. By using these techniques, it is possible to apply the coating layer(s) on the membrane more efficiently.

In one embodiment of the fifth aspect, the step of forming is performed by using at least one of particle/fluid bombardment, etching and laser burning. These techniques may allow one to form the through pores in the whole cell culture membrane structure more efficiently.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a schematic perspective view of a cell culture membrane structure in accordance with a first exemplary embodiment;
FIG. 2 shows a flowchart of a method for producing the cell culture membrane structure shown in FIG. 1;
FIG. 3 explains how to apply a dual coating on the structure shown in FIG. 1 by using a spin coating process in the method shown in FIG. 2;
FIG. 4 shows a schematic perspective view of a cell culture membrane structure in accordance with a second exemplary embodiment;
FIG. 5 shows a flowchart of a method for producing the cell culture membrane structure shown in FIG. 4;
FIG. 6 shows a schematic perspective view of a cell culture plate in accordance with one exemplary embodiment;
FIG. 7 shows a schematic cross-sectional view of one well of the cell culture plate shown in FIG. 6, as taken along the line A-A of FIG. 6;
FIG. 8 shows a block-scheme of a microfluidic device or chip in accordance with a first exemplary embodiment;
FIG. 9 shows a block-scheme of a microfluidic device or chip in accordance with a second exemplary embodiment.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, the structure, devices and/or methods disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above" "below", "upper", "lower", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first side of a membrane discussed below could be called a second side of the membrane, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

The exemplary embodiments disclosed herein refer to a porous membrane structure that may be used as a cell culture support adapted for specific cells to be grown thereon. The porous membrane structure comprises a membrane having through pores formed therein and at least one coating layer provided on the membrane such that the through pores remain open. Said adaptation is provided by selecting membrane and coating materials based on at least one type of cells to be grown on the at least one coating layer. By so doing, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells.

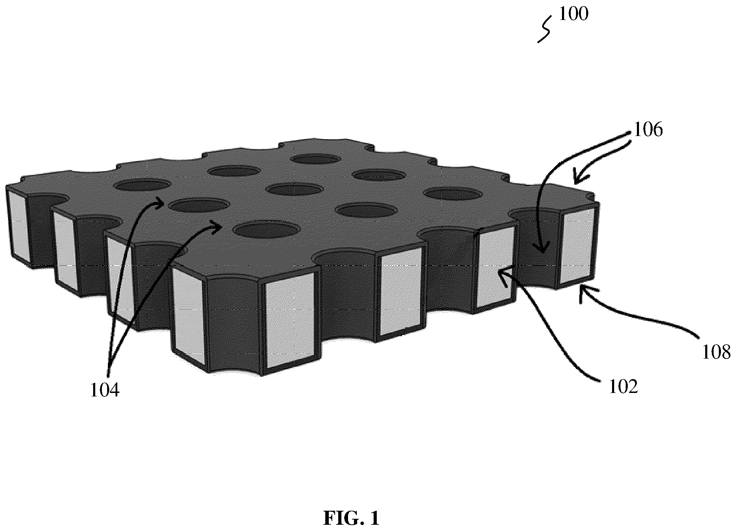

FIG. 1 shows a schematic perspective view of a cell culture membrane structure 100 in accordance with a first exemplary embodiment. As shown in FIG. 1, the structure 100 comprises a membrane 102 with through pores 104 formed therein, a first coating layer 106 provided on a first (top) side of the membrane 102, and a second coating layer 108 provided on a second (bottom) side of the membrane 102. The first and second coating layers 106 and 108 are provided on the first and second sides of the membrane 102 such that the through pores 104 remain open. The membrane 102 may be made of one of the following materials: polypropylene, polycarbonate, polyethersulfone, polyethylene terephthalate, polyimide, polytetrafluoroethylene (PTFE), silicone (e.g., commercially available sylgard, roomtemperature-vulcanizing (RTV) silicone, elastosil), and silicon nitride. The first and second coating layers 106 and 108 may be made of the same coating material or different coating materials, depending on particular applications (in particular, depending on certain cells to be grown on the first and second coating layers 106 and 108). The coating material for each of the first and second coating layers 106 and 108 may be selected from one of the following materials: silicone, polyacrylamide, PEG polymer, acrylamide, gelatin derivative, and nanocellulose.

In one other embodiment, one of the first and second coating layers 106 and 108 may be not provided on the membrane 102 if it is required to study cell behavior only on the first or second side of the membrane 102. Thus, the presence of the two coating layers 106 and 108 should not be construed as any limitation of the present disclosure. Moreover, the first and second coating layers 106 and 108 may have the same or different thicknesses, depending on particular applications. In one embodiment, the thicknesses of the first and second coating layers 106 and 108 may vary from 200 nanometers to 50 micrometers. In a preferred embodiment, the thicknesses of the first and second coating layers 106 and 108 vary from 500 nanometers to 3000 nanometers. At the same time, care should be taken when selecting the thicknesses of the first and second coating layers 106 and 108, since an increase in the thickness of the whole structure 100 impacts the interaction of the cells to be grown on the opposite first and second sides of the membrane 102.

As an addition or alternative, the first and second coating layers 106 and 108 may differ from each other in stiffness. For example, the first coating layer 106 may be made of low-stiffness silicone to provide a low-cell attachment surface for osteoblast cell culture, while the second coating layer 108 may be made of high-stiffness silicone for endothelial cell culture. Such a membrane structure 100 will allow one to implement a specified bone-marrow-on-chip model.

As for the through pores 104, their circular cross-section shape shown in FIG. 1 should not also be construed as any limitation of the present disclosure. In some embodiments, the cross-section shape of the through pores 104 may be triangular, rectangular, square, oval, or polygonal (e.g., hexagonal). The polygonal cross-section shape of the through pores 104 may refer to a convex or concave polygon. In some other embodiments, the through pores 104 may have different cross-section shapes, such as any combination of two or more of the above-mentioned cross-section shapes (e.g., the combination of circular and square cross-section shapes).

In one embodiment, each of the through pores 104 may have a pore size varying within a predefined range of values, and a spacing (pitch) between the through pores 104 may vary within the same predefined range of values. For example, the pore size and spacing may both vary from 200 nanometers to 30 micrometers. In general, the predefined range of values for the pore size and the spacing between the through pores 104 is selected based on certain cells to be grown on the first and second coating layers 106 and 108. It should be apparent that a pore density will depend on the pore size and spacing selected depending on particular applications. The pore density may, for example, be from 10³ to 10⁷ pores per millimeter square. Additionally, the pore sizes may be more or less than sizes of the cells to be grown on the first and second coating layers 106 and 108. For example, if the pore size is less than the cell size, then it is possible to prevent the cells from migrating between the first and second sides of the membrane 102.

In one embodiment in which cells of two different types are to be initially grown on the first coating layer 106 or the second coating layer 108 (i.e. on the same side of the membrane 102), each of the through pores 104 may have a pore size varying within a predefined range of values which is selected (e.g., based on sizes and/or elastic properties of the cells of the two types) such that the through pores 104 pass or filter the cells of one of the two types therethrough, while retaining (i.e. blocking) the cells of another of the two types. In this embodiment, such through pores 104 may be also spaced from each other by a distance varying within the same predefined range of values. By having such through pores 104 in the membrane 102, it is possible to sort out the cells according to their types.

In one embodiment, the structure 100 may further comprise chemical agents and/or biomolecules bonded onto at least one of the first and second coating layers 106 and 108. The chemical agents may be represented by antifouling agents, such, for example, as pluronics or mono/polyethylene glycol (MEG/PEG) polymers, or lipids. The biomolecules may be represented by antigens, proteins, carbohydrate, antibody molecules, etc. By using such chemical agents and/or biomolecules, it is possible to provide an even larger spectrum of functionality of the structure 100.

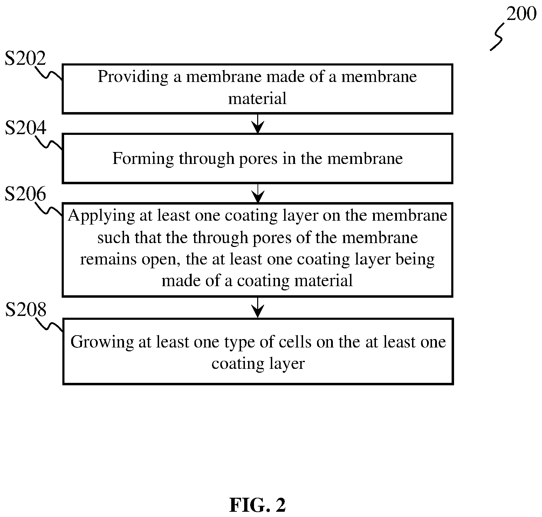

FIG. 2 shows a flowchart of a method 200 for producing the cell culture membrane structure 100. As shown in FIG. 2, the method 200 starts with a step S202, in which the membrane 102 made of one of the above-mentioned membrane materials is provided. In other words, the step S202 consists in fabricating the membrane 102 of the selected membrane material. Then, the method 200 proceeds to a step S204, in which the through pores 104 are formed in the membrane 102. After that, the method 200 goes on to a step S206, in which at least one of the first and second coating layers 106 and 108 is applied on the membrane 102. The first and/or second coating layers 106 and 108 should be applied such that the through pores 104 of the membrane 102 remain open. As noted earlier, the membrane and coating materials are selected based on the cells to be grown on the first and/or second coating layers 106 and 108.

As also shown in FIG. 2, the method 200 comprises a step S208, in which the cells of one or more types are grown on at least one of the first and second layers 106 and 108. However, the step S208 is optional and may be omitted. In particular, the method 200 may end up with the step S206, thereby providing the cell culture membrane structure 100 manufactured as a roll or sheet with no cells grown thereon. The roll or sheet of the structure 100 may be provided to an end user who may then grow or culture the cells of desired type(s) (e.g., yeast cells, insect cells, mammalian cells, bacterial cells, tissue cells, etc.) on the structure 100 (e.g., on the first and second coating layers 106 and 108), for example, for the purpose of cell behavior study.

In one embodiment, the step S204 may be performed by using at least one of particle/fluid bombardment, etching and laser burning. These techniques may allow the through pores 104 to be formed more efficiently.

In one embodiment, the step S206 may consist in applying the first coating layer 106 and/or the second coating layer 108 on the membrane 102 in a continuous manner. Then, the through pores 104 of the membrane 102 may be cleared by using etching and/or air or liquid pressurizing techniques. By using these techniques, it is possible to remove the coating material from the through pores 104 more efficiently.

In one embodiment, the first coating layer 106 and/or the second coating layer 108 may be applied on the membrane 102 by using one of spin coating, blade coating, spray coating, immersion coating, brush coating, stamping, and electrospraying. It should be noted that clearing techniques, such as etching and/or air or liquid pressurizing, may not be required in case of using the spin coating process because spinning or centrifugal forces occurring in the spin coating process should clear the through pores 104 by their own.

In one embodiment, the method 200 may comprise a further step, in which electrically conductive materials or electrodes are printed or etched onto the membrane 102 before the step S206. By using such electrodes, it is possible to study cell behavior in the presence of electric current.

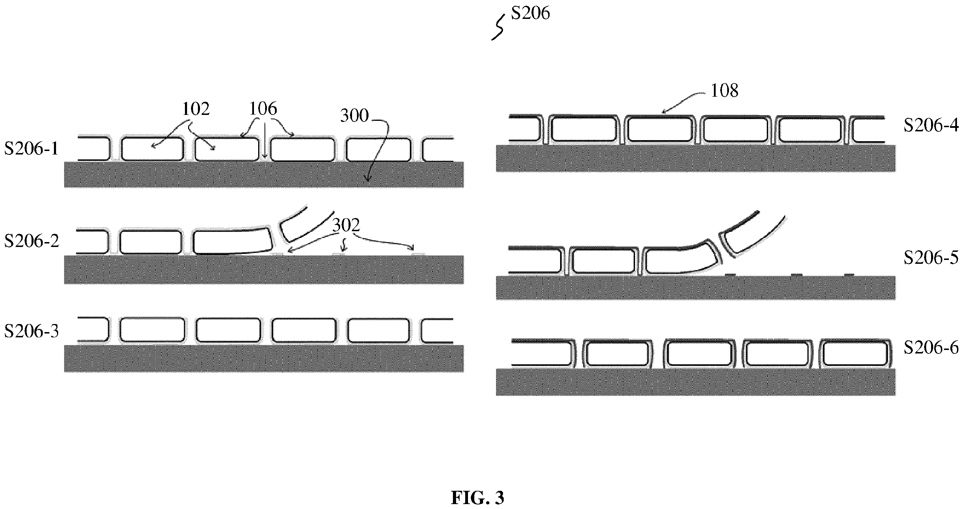

FIG. 3 explains how to apply a dual coating (i.e. the first and second coating layers 106 and 108) on the membrane 102 by using the spin coating process in the step S206 of the method 200. In this case, the step S206 comprises substeps S206-1 - S206-6. Let us assume that the first coating layer 106 is made of a first coating material (colored in light gray in FIG. 3), while the second coating layer 108 is made of a different second coating material (colored in black in FIG. 3). At first, in the substep S206-1, the membrane 102 is arranged on a working surface 300 (e.g., a flat wafer or substrate), and the first (top) side of the membrane 102 is spin-coated with the first coating material, thereby forming the first coating layer 106. Since the first coating layer 106 is provided as the thin meniscus of the first coating material on the membrane 102, a curing process may be also applied in the substep S206-1, such as heating or exposure to UV-light. Further, in the substep S206-2, the membrane 102 with the first coating layer 106 is peeled off the working surface 300. Due to the presence of the through pores 104 in the membrane 102, said peeling-off may lead to residual coating sticks 302 left on the working surface 300. The residual coating sticks 302 should be removed from the working surface 300. In the next substep S206-3, the membrane 102 is turned or flipped to access the second (bottom) side of the membrane 102 and arranged on the working surface 300. After that, the second side of the membrane 102 is spin-coated with the second coating material in the substep S206-4, thereby forming the second coating layer 108. Again, the second coating layer 108 may be also cured in the substep S206-4. The dual-coat membrane 102 or, in other words, the structure 100 is further peeled off the working surface 300, thereby leaving residual coating sticks of the second coating material (which may be removed if the working surface 300 is intended to be used further). In the last substep S206-6, the structure 100 is ready for further applications or processing.

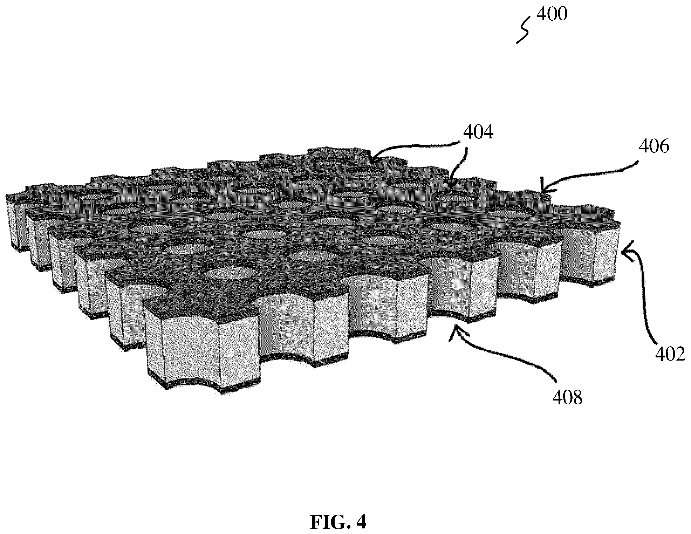

FIG. 4 shows a schematic perspective view of a cell culture membrane structure 400 in accordance with a second exemplary embodiment. As shown in FIG. 4, the structure 400 comprises a membrane 402 with through pores 404 formed therein, a first coating layer 406 provided on a first (top) side of the membrane 402, and a second coating layer 408 provided on a second (bottom) side of the membrane 402. The first and second coating layers 406 and 408 are provided on the first and second sides of the membrane 402 such that the through pores 404 remain open. The membrane 402 and the coating layers 406 and 408 may be made of the same materials which are mentioned above with reference to the membrane 102 and the coating layers 106 and 108, respectively, which are included in the structure 100 shown in FIG. 1. Similar to the coating layers 106 and 108, the coating layers 406 and 408 may be made of the same coating material or different coating materials and/or may have the same or different thicknesses, and/or one of the coating layers 406 and 408 may be not provided on the membrane 402, depending on particular applications (in particular, depending on certain cells to be grown thereon). Moreover, the through pores 404 may be implemented in the same manner as the through pores 104 in the structure 100. As can be seen from FIG. 4, the structure 400 differs from the structure 100 in that it has no coating layer or material applied on the inner surface of each through pore 404, which is caused by the specifics of its production, as will be explained below.

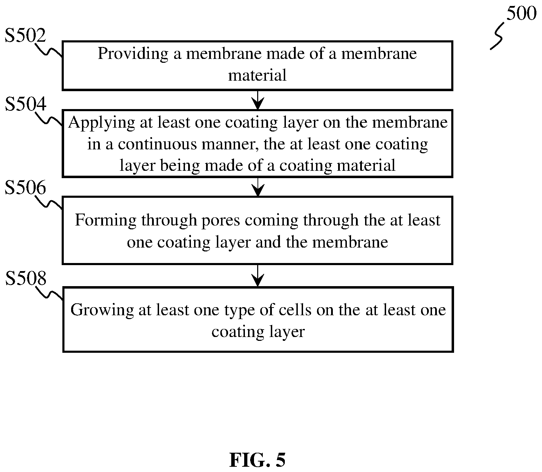

FIG. 5 shows a flowchart of a method 500 for producing the cell culture membrane structure 400. As shown in FIG. 5, the method 500 starts with a step S502, in which the membrane 402 made of one of the above-mentioned membrane materials is provided. In other words, the step S502 consists in fabricating the membrane 402 of the selected membrane material. Then, contrary to the method 200, the method 500 proceeds to a step S504, in which at least one of the first and second coating layers 406 and 408 is applied on the membrane 402 in a continuous manner. The step S504 may be performed by using one of spin coating, blade coating, spray coating, immersion coating, brush coating, stamping, and electrospraying. After that, the method 500 goes on to a step S506, in which the through pores 404 are formed in the membrane 402. Since the through pores 404 are formed after the first coating layer 406 and/or the second coating layer 408 are applied on the membrane 402, there is no coating layer on the inner surface of each through pore 404. As noted earlier, the membrane and coating materials are selected based on the cells to be grown on the first and/or second coating layers 406 and 408.

As also shown in FIG. 5, the method 500 comprises a step S508, in which the cells of one or more types are grown on at least one of the first and second layer 406 and 408. However, similar to the step S208 of the method 200, the step S508 of the method 500 is optional and may be omitted for the same reasons. In other words, the method 500 may end up with the step S506, thereby providing the cell culture membrane structure 400 manufactured as a roll or sheet with no cells grown thereon. The roll or sheet of the structure 400 may be provided to an end user who may then grow or culture the cells of desired type(s) (e.g., mammalian cells, bacterial cells, tissue cells, etc.) on the structure 400 (e.g., on the first and second coating layers 406 and 408), for example, for the purpose of cell behavior study.

It should be also noted that the step S506 of the method 500 may be implemented similar to the step S204 of the method 200. That is, the through pores 404 may be formed by using at least one of particle/fluid bombardment, etching and laser burning.

In one embodiment, similar to the method 200, the method 500 may comprise a further step, in which electrically conductive materials or electrodes are printed or etched onto the membrane 402 before the step S504. By using such electrodes, it is possible to study cell behavior in the presence of electric current.

Let us now give two non-limitative practical examples of how to apply a dual coating on a desired membrane by using the spin-coating process, depending on particular applications.

It should be noted that the dual coating discussed in the two practical examples may be applied either in the step S206 of the method 200 or in the step S504 of the method 500, meaning that the desired membrane may correspond to the membrane 102 or 402, respectively. In other words, the dual coating discussed in the two practical examples may be applied after or before through holes are formed in the desired membrane.

### Practical example 1

In this practical example, it is assumed that a first coating layer (i.e. the coating layer 106 or 406) is made of low-stiffness silicone to provide a low-cell attachment surface for osteoblast cell culture, while the second coating layer (i.e. the coating layer 108 or 408) is made of high-stiffness silicone for endothelial cell culture. Such a dual-coat membrane structure allows one to implement a specified bone-marrow-on-chip model.

The dual-coating process itself (i.e. the step S206 or S504) may be performed as follows:
1. The membrane having a thickness of 3 microns is fixed on a silicon wafer (which serves, e.g., as the working surface 300) by a double-sided adhesive.
2. The first coating layer consisting of a 10% solution of silicone in hexane is spin-coated on the membrane, with the spin-coating parameters being as follows: spin speed - 2000 rpm, time - 90 seconds.
2. The membrane is peeled off from the silicon wafer by dissolving the adhesive in hot water.
3. The membrane is carefully placed on the same silicon wafer or a new silicon wafer with its uncoated side facing up.
4. The second coating layer consisting of a 50% solution of silicone in hexane is then spin-coated on the membrane, with the spin-coating parameters being as follows: spin speed - 5000 rpm, time - 120 seconds.
5. The membrane now coated on both sides is peeled off from the silicon wafer and placed in an oven set at 85 degrees Celsius. The membrane is cured for 1 hour.

### Practical example 2

This practical example relates to a gut-on-chip model, in which a gut compartment of a chip is assumed to require that the desired membrane has one (e.g., top) low-adhesion surface to allow for a curvy gut epithelia culture and another (e.g., bottom) high-adhesion surface to allow for endothelial cell attachment.

Given this, the dual-coating process itself (i.e. the step S206 or S504) may be performed as follows:
1. The membrane having a thickness of 3 microns is fixed on a silicon wafer (which serves, e.g., as the working surface 300) by a double-sided adhesive.
2. The first coating layer consisting of a 4% solution of pluronic in water is spin-coated on thr membrane, with the spin-coating parameters being as follows: spin speed - 100 rpm, time - 90 seconds. This yields the low-adhesion surface for the gut epithelia culture.
3. The membrane is peeled off from the silicon wafer by dissolving the adhesive in hot water.
4. The membrane is carefully placed on the same silicon wafer or a new silicon wafer with its uncoated side facing up.
5. The second coating layer consisting of a 50% solution of silicone in hexane is then spin-coated on the membrane, with the spin-coating parameters being as follows: spin speed - 5000 rpm, time - 120 seconds. This yields a silicone coating which may then be coated with a 100 ug/mL fibronectin solution in water for the endothelial cell attachment.
6. The membrane now coated on both sides is peeled off from the silicon wafer and placed in an oven set at 85 degrees Celsius. The membrane is cured for 1 hour.

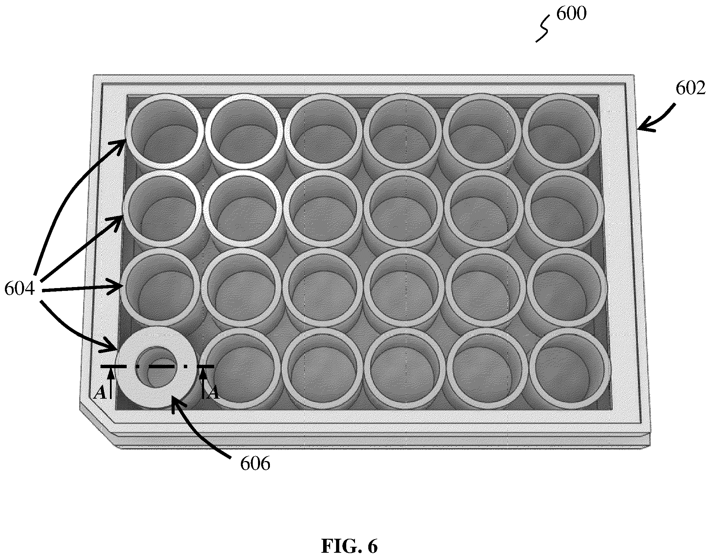

FIG. 6 shows a schematic perspective view of a cell culture plate 600 in accordance with one exemplary embodiment. As shown in FIG. 6, the cell culture plate 600 comprises a rigid housing 602 encompassing a plurality of wells 604. Each well 604 is shaped as a cylinder and is provided with a cell culture media and a replaceable insert 606 configured to be immersed into the cell culture media. Each of the inserts 606 may comprise the cell culture membrane structure 100 or the cell culture membrane structure 400. The cell culture plate 600 makes it possible to grow cells of desired type(s) on a large amount of cell culture membrane structures, like the structures 100 and/or 400, simultaneously and under the same growing conditions. It should be apparent to those skilled in the art that the number, shape and spacing of the wells 604 shown in FIG. 6 are for illustrative purposes only and should not be construed as any limitation of the present disclosure.

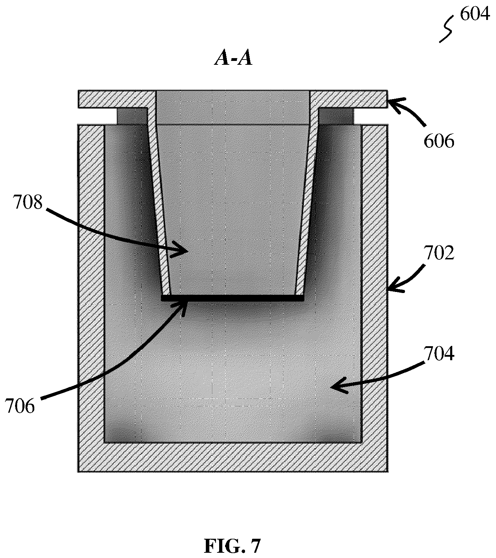

FIG. 7 shows a schematic cross-sectional view of one well 604 of the cell culture plate 600, as taken along the line A-A of FIG. 6. As shown in FIG. 7, the well 604 comprises a body 702 with an inner cavity 704 which is filled with the cell culture media. The insert 606 is immersed into the inner cavity 704 and has a cell culture membrane structure 706 attached to the bottom of the insert 606. The cell culture membrane structure 706 may be implemented as the cell culture membrane structure 100 or 400, depending on particular applications. The insert 606 is provided with an inner cavity 708. The inner cavity 708 may be used, for example, to place different biological objects (e.g., an organoid or spheroid) on the top side of the cell culture membrane structure 706 for research purposes.

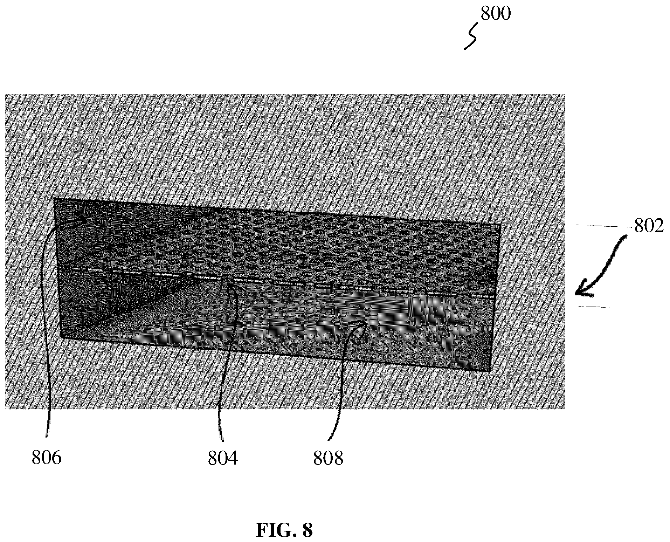

FIG. 8 shows a block-scheme of a microfluidic device or chip 800 in accordance with a first exemplary embodiment. As shown in FIG. 8, the device 800 comprises a rigid housing 802 and a microfluidic flow channel which is divided by a cell culture membrane structure 804 into two microfluidic flow subchannels 806 and 808. The cell culture membrane structure 804 may be implemented as the cell culture membrane structure 100 or 400, depending on particular applications. Each of the microfluidic flow subchannels 806 and 808 may be used to pass a certain fluid or cell culture media in the same or opposite directions. The cell culture membrane structure 804 may initially have certain cells grown thereon, or such cells may be deposited on the cell culture membrane structure 804 when the fluid or cell culture media is perfused through the microfluidic flow subchannels 806 and 808. In some application scenarios, the microfluidic flow subchannels 806 and 808 may mimic blood vessels. It should be noted that the sizes of the microfluidic flow subchannels 806 and 808 and the cell culture membrane structure 804 are exaggerated for convenience. Moreover, it should be apparent to those skilled in the art that the device 800 may comprise more than one microfluidic flow channel, if required, and each microfluidic flow channel included in the device 800 may be divided by the cell culture membrane structure 100 or 400 into two equal or different microfluidic flow subchannels.

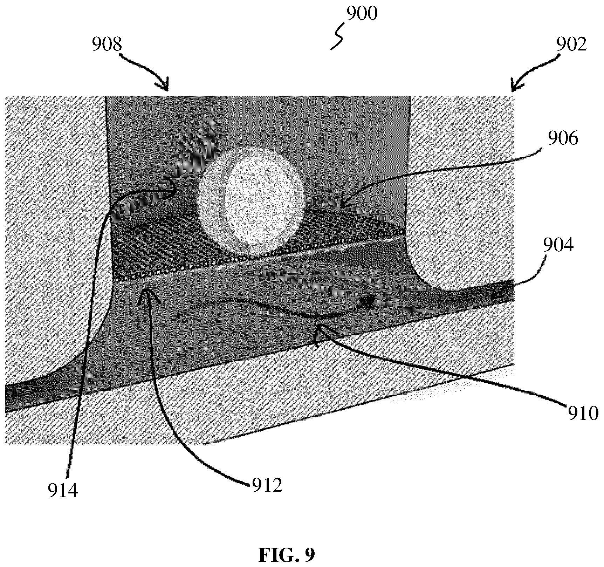

FIG. 9 shows a block-scheme of a microfluidic device or chip 900 in accordance with a second exemplary embodiment. As shown in FIG. 9, the device 900 comprises a rigid housing 902, a microfluidic flow channel 904, and a cell culture membrane structure 906 brought into contact with the microfluidic flow channel 904. The cell culture membrane structure 906 may be implemented as the cell culture membrane structure 100 or 400, depending on particular applications. The device 900 may also comprise an optional top-loaded chamber 908 used to bring the cell culture membrane structure 906 into contact with an additional cell culture media or biological objects. The device 900 may operate as follows. A cell suspension 910 is perfused through the microfluidic flow channel 904, thereby depositing cells therein. The device 900 is then inverted and a gravity force causes the cells to settle on the cell culture membrane structure 906. The cells are cultured on the cell culture membrane structure 906 until they are attached thereto (which takes a minimum of 1 hour). Once the cells form a desired layer 912 on the cell culture membrane structure 906, the device 900 is inverted again. After that, another cell culture media is added through the top-loaded chamber 908 to the other side of the cell culture membrane structure 906. An organoid 914 or other biological object may be optionally placed into the device 900 (on the top side of the cell culture membrane structure 906). The co-culture is cultivated for a desired period of time while the bottom microfluidic flow channel 904 is perfused, and cell behavior is then observed. During this period, the device 900 may be challenged with different chemicals, culture conditions, drugs, etc. Moreover, additional cell types may be added onto the organoid or into the bottom microfluidic flow channel 904. Although the device 900 is shown to have one microfluidic flow channel 904 and/or one top-loaded chamber 908, this should not be construed as any limitation of the present disclosure; in some other embodiments, the device 900 may be provided with multiple microfluidic flow channels 904 and multiple top-loaded chambers 908 each connected to one or more of the microfluidic flow channels 904.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A cell culture membrane structure comprising:
a membrane made of a membrane material, the membrane having through pores formed therein; and
at least one coating layer provided on the membrane such that the through pores of the membrane remain open, the at least one coating layer being made of a coating material;
wherein the membrane material and the coating material are selected based on at least one type of cells to be grown on the at least one coating layer.

2. The structure of claim 1, further comprising chemical agents and/or biomolecules bonded onto the at least one coating layer.

3. The structure of claim 1 or 2, wherein the at least one coating layer comprises a first coating layer provided on a first side of the membrane and a second coating layer provided on a second opposite side of the membrane.

4. The structure of claim 3, wherein the coating material is the same or different for each of the first coating layer and the second coating layer.

5. The structure of claim 3 or 4, wherein each of the first coating layer and the second coating layer has a different thickness and/or stiffness.

6. The structure of any one of claims 3 to 5, wherein the at least one type of cells comprises a first type of cells to be grown on the first coating layer and a second type of cells to be grown on the second coating layer.

7. The structure of any one of claims 1 to 6, wherein each of the through pores has a pore size varying within a predefined range of values, and wherein the through pores are spaced from each other by a distance varying within the predefined range of values, the predefined range of values being selected based on the at least one type of cells to be grown on the at least one coating layer.

8. The structure of claim 7, wherein the pore sizes of the through pores are selected to be less than cell sizes of the at least one type of cells to be grown on the at least one coating layer.

9. The structure of any one of claims 3 to 5, wherein the at least one type of cells comprises a first type of cells and a second type of cells which are both to be grown on one of the first coating layer and the second coating layer.

10. The structure of claim 9, wherein each of the through pores has a pore size varying within a predefined range of values, the through pores being spaced from each other by a distance varying within the predefined range of values, and wherein the predefined range of values is selected such that the through pores are configured to pass one of the first type of cells and the second type of cells therethrough and retain another of the first type of cells and the second type of cells.

11. The structure of claim 10, wherein the predefined range of values is selected based on elastic properties of the first type of cells and the second type of cells.

12. The structure of any one of claims 1 to 11, wherein the membrane material is selected from one of polypropylene, polycarbonate, polyethersulfone, polyethylene terephthalate, polyimide, polytetrafluoroethylene, silicone, silicon nitride.

13. The structure of any one of claims 1 to 12, wherein the coating material is selected from one or more of silicone, polyacrylamide, PEG polymer, acrylamide, gelatin derivative, nanocellulose.

14. A cell culture plate comprising:
a housing encompassing a plurality of wells, each well being provided with a cell culture media and a replaceable insert configured to be immersed into the cell culture media, the insert comprising the cell culture membrane structure according to any one of claims 1 to 13.

15. A microfluidic device comprising:
a housing having at least one microfluidic flow channel formed therein, each of the at least one microfluidic flow channel being configured to pass a fluid or cell culture media therethrough; and
at least one cell culture membrane structure according to any one of claims 1 to 13, each of the at least one cell culture structure being in contact with one of the at least one microfluidic flow channel.

16. The device of claim 15, wherein the housing further comprises at least one top-loaded chamber, and wherein each of the at least one top-loaded chamber is connected to one of the at least one microfluidic flow channel via one of the at least one cell culture membrane structure.

17. The device of claim 15, wherein each of the at least one microfluidic flow channel is divided by one of the at least one cell culture membrane structure into two microfluidic flow subchannels.

18. A method for producing a cell culture membrane structure, comprising:
providing a membrane made of a membrane material,
forming through pores in the membrane; and
applying at least one coating layer on the membrane such that the through pores of the membrane remain open, the at least one coating layer being made of a coating material;
wherein the membrane material and the coating material are selected based on at least one type of cells to be grown on the at least one coating layer.

19. A method for producing a cell culture membrane structure, comprising:
providing a membrane made of a membrane material,
applying at least one coating layer on the membrane in a continuous manner, the at least one coating layer being made of a coating material; and
forming through pores coming through the at least one coating layer and the membrane;
wherein the membrane material and the coating material are selected based on at least one type of cells to be grown on the at least one coating layer.
